Europäisches Patentamt

European Patent Office

Office européen des brevets

(11) Publication number: **0 189 861**
**A2**

# (12) EUROPEAN PATENT APPLICATION

(21) Application number: 86100939.7

(51) Int. Cl.⁴: **A 61 K 47/00**

(22) Date of filing: 24.01.86

(30) Priority: 26.01.85 JP 11767/85
02.05.85 JP 93821/85
14.05.85 JP 100483/85
14.05.85 JP 100484/85
22.05.85 JP 108332/85
23.05.85 JP 109279/85
13.09.85 JP 201738/85

(43) Date of publication of application:
06.08.86 Bulletin 86/32

(84) Designated Contracting States:
CH DE FR GB IT LI

(71) Applicant: SHOWA DENKO K.K.
13-9, Shiba Daimon 1-chome
Minato-ku, Tokyo 105(JP)

(72) Inventor: Satoh, Motoaki
Showa Denko Kabushiki Kaisha Seikagaku Kenkyusho
24-25, Tamagawa 2-chome Oota-ku Tokyo(JP)

(72) Inventor: Sakai, Yasuyuki
Showa Denko Kabushiki Kaisha Seikagaku Kenkyusho
24-25, Tamagawa 2-chome Oota-ku Tokyo(JP)

(72) Inventor: Shishikura, Takashi
Showa Denko Kabushiki Kaisha Seikagaku Kenkyusho
24-25, Tamagawa 2-chome Oota-ku Tokyo(JP)

(72) Inventor: Yokoi, Hirotsugu
Showa Denko Kabushiki Kaisha Seikagaku Kenkyusho
24-25, Tamagawa 2-chome Oota-ku Tokyo(JP)

(72) Inventor: Ishikura, Toyoaki
Showa Denko Kabushiki Kaisha Seikagaku Kenkyusho
24-25, Tamagawa 2-chome Oota-ku Tokyo(JP)

(72) Inventor: Sugimori, Hiroko
Showa Denko Kabushiki Kaisha Seikagaku Kenkyusho
24-25, Tamagawa 2-chome Oota-ku Tokyo(JP)

(72) Inventor: Ebisawa, Hisashi
Showa Denko Kabushiki Kaisha Seikagaku Kenkyusho
24-25, Tamagawa 2-chome Oota-ku Tokyo(JP)

(72) Inventor: Takahashi, Michiyo
Showa Denko Kabushiki Kaisha Seikagaku Kenkyusho
24-25, Tamagawa 2-chome Oota-ku Tokyo(JP)

(72) Inventor: Hasegawa, Yuko
Showa Denko Kabushiki Kaisha Seikagaku Kenkyusho
24-25, Tamagawa 2-chome Oota-ku Tokyo(JP)

(74) Representative: Strehl, Schübel-Hopf, Groening, Schulz
Widenmayerstrasse 17 Postfach 22 03 45
D-8000 München 22(DE)

(54) Percutaneous absorption accelerator for ionic water-soluble medicine.

(57) A percutaneous absorption accelerator for ionic water-soluble medicines comprising at least one member selected from the group consisting of ionic oil-soluble substances and their salts, amphoteric surfactants, and nonionic substances. These substances can effectively and efficiently accelerate the desired percutaneous absorption of ionic water-soluble medicines.

0189861

# PERCUTANEOUS ABSORPTION ACCELERATOR
## FOR IONIC WATER-SOLUBLE MEDICINE

BACKGROUND OF THE INVENTION

1.   Field of the Invention

The present invention relates to a percutaneous or transdermal absorption accelerator for ionic water-soluble medicines or pharmaceutically active agents. More specifically, it relates to the percutaneous absorption acceleration of ionic water-soluble medicines including cationic and anionic water-soluble medicines by incorporating ionic oil-soluble substances and their salts, amphoteric surfactants, and/or nonionic substances into transdermal therapeutic preparations containing the ionic water-soluble medicines.

2.   Description of the Related Art

It is well-known in the art that transdermal therapeutic preparations are used for the purposes of locally effecting antiphlogistic and analgesic activities (e.g., anti-inflammatory) or dermatopathy treatment (e.g., adrenocortical hormones or anti-fungal agents). These percutaneous administrations are expected to be advantegous when compared with other conventional medicine administration methods such as intraoral, intravenous, intramuscular, and intrarectal administration methods, in, for example, the sustained absorption of medicines, the prolongation of pharmacological activities, the wide absorption area, the reduction of administration times and side effects, the simplification of treatments, the avoidance of a refusal to take medicines, and an improvement in the biological utilization efficiency of medicines (see, for example, Drug Dev. Ind. Pharm. 9(4), 725, 1983).

From the above-mentioned standpoint, various attempts have been made to develop transdermal therapeutic systems capable of systemically effecting pharmacological activities. For example, (1) anti-kinesia agents (e.g., scopolamine) and (2) circulating system agents (e.g., nitroglycerin, isosorbide dinitrate, clonidine, propranol) are commercially available or are under clinical evaluation tests (see, for example, Pharmacol., Therap., 13(1), 149, 1981; Gekkan Yakuji, 26(3), 155, 1984; Yakkyoku, 34(11), 1509, 1983; Rinsho Yakuri, 13, 463, 1982).

In order to develop transdermal therapeutic preparations capable of systemically effecting pharmacological activities, it is essential for medicines to be effectively and sustainingly absorbed percutaneously or transdermally. Regarding the percutaneous absorption of medicines, it is generally admitted in the art that the properties, especially lipid-solubilities, of medicines are the controlling factors and, therefore, all medicines contained in the previously developed transdermal therapeutic preparations have appropriate lipid-solubilities (see, for example, Drug Dev. Ind. Pharm., 9(4), 671, 1983).

On the other hand, many medicines now in clinical use are water-soluble and, therefore, it is clear that, if these water-soluble medicines can be percutaneously or transdermally absorbed, the clinical usefulness will be great when compared to the conventional administration routes. However, it is disclosed in, for example, Drug Dev. Ind. Pharm. 9(4), 627 (1983) and Shinseizai Kaihatsu System Sogo Gijutsu, 221 (1985) that corneal layers of epidermis become strong barriers against the percutaneous absorption of water-soluble medicines. The inventors are aware of only very limited reports regarding the percutaneous absorption of water-soluble medicines. For example, the following reports are known.

(1) An aqueous and/or alcoholic solution of water-soluble medicines is dissolved in an adhesive polymer solution containing, as a main component, alkyl methacrylates and film type preparations are prepared therefrom (see Japanese Unexamined Patent Publication (Kokai) No. 59-164715).

(2) Transdermal therapeutic preparations containing a water-soluble antihistamine, isothipendyl hydrochloride and water-soluble basic substances or nonionic surfactants (see Japanese Unexamined Patent Publication (Kokai) Nos. 59-51212 and 59-98014).

(3) Transdermal therapeutic preparations in the form of a tape containing hydrophillic medicines and organic acids or metal salts (e.g., Mg, Ca, Zn, or Al salts) thereof (see Japanese Unexamined Patent Publication (Kokai) No. 59-164714).

(4) Transdermal therapeutic preparations containing sodium polyacrylates, aluminum hydroxide, and percutaneously absorbable medicines (see Japanese Unexamined Patent Publication (Kokai) No. 59-25320).

However, all of the above-mentioned techniques are intended to improve the stability or solubility of the medicines during the manufacture thereof, the sustained releasability of the medicines from the preparations, and the complicated or troublesome manufacturing operations. Thus, sufficient explanation or discussion is not given in the above-mentioned publications as to the percutaneous absorption acceleration effects of medicines through the corneal layers of epidermis, or the pharmacological effects of the medicines.

On the other hand, in order to improve the percutaneous absorption of medicines, it is proposed in Japanese Unexamined Patent Publication (Kokai) Nos. 51-32724 and 52-83914 that anionic and/or amphoteric surfactants exhibiting a pH of 4 to 8 in an aqueous solvent are included in transdermal therapeutic preparations. However, these publications only teach the percutaneous absorption acceleration effects of extremely slightly water-soluble medicines. These publications do not teach the percutaneous absorption acceleration of water-soluble medicines.  In addition, it is reported in, for example, Drug Dev. Ind. Pharm., 9(4), 627 (1983) that dimethylsulfoxide and dimethylformamide can reduce the barrier action of the corneal layers of epidermis and, therefore, increase the percutaneous absorption capability of medicines.  However, it is believed that this phenomenon is caused by denaturing the corneal layers of epidermis with dimethylsulfoxide or dimethylformamide.  This is, of course, not preferable from the point of view of skin irritation and these preparations cannot be applied directly to the skin, especially when a long term application is required.

Furthermore, it is reported in J. Pharm. Pharmacol., 36, 22 (1984) that long-chain amines accelerate the penetration abilities of sodium salicylate through fat and oil membranes in vitro.  However, although this shows an improvement in the fat and oil partition coefficients of water-soluble medicines as known in phase transfer reactions, there is no teaching of the percutaneous absorption acceleration effects in the living bodies.

Thus, it has not been established yet in the art to accelerate the absorption of water-soluble medicines percutaneously or transdermally in an amount sufficient to exhibit useful pharmacological activities.

SUMMARY OF THE INVENTION

Accordingly, the objects of the present invention are to eliminate the above-mentioned disadvantages of the prior art and to provide a percutaneous absorption accelerator for ionic water-soluble medicines.

Other objects and advantages of the present invention will be apparent from the following description.

In accordance with the present invention, there is provided a percutaneous absorption accelerator for ionic water-soluble medicines comprising at least one member selected from the group consisting of ionic oil-soluble substances and their salts, amphoteric surfactants, and nonionic substances.

DESCRIPTION OF THE PREFERRED EMBODIMENT

According to the present invention, the percutaneous absorption of cationic or anionic water-soluble medicines is adyantegeously accelerated by the use of ionic oil-soluble substances and their salts amphoteric surfactsnts, and/or nonionic substances.

The cationic water-soluble medicines usable in the present invention preferably include those having a cationic group such as an amino group, a pKa of 7.0 or more, and a solubility such that 1 g of the medicine can be dissolved in an amount of less than 10 ml of water at 25°C. Typical examples of such medicines are medicines for the circulating system such as acebutolol hydrochloride, alprenolol hydrochloride, metoprolol tartrate, indenolol hydrochloride, oxprenolol hydrochloride, bufetolol hydrochloride, bunitrolol hydrochloride, trimetazidine hydrochloride, disopyramide phosphate, procainamide hydrochloride, diltiazem hydrochloride, guanethidine sulfate, betanidine sulfate, mecamylamine hydrochloride, norfenefrine hydrochloride, etilefrine hydrochloride, and octopamine hydrochloride; psychotropic medicines such as fluphenazine dihydrochloride, amitriptyline hydrochloride, imipramine hydrochloride, opipramol hydrochloride, and chlorpromazine hydrochloride; antiinflammatory analgesic medicines such as tramadol hydrochloride and benzydamine hydrochloride; antispasmodic medicines such as acamylophenine hydrochloride, trospium chloride, valethamate bromide, scopolamine butylbromide, prifinium bromide, diponium bromide, and N-methylscopolamine methylsulfate; anethetic medicines such as ketamine hydrochloride, tetracaine hydrochloride, cocaine hydrochloride, dibucaine hydrochloride, procaine hydrochloride, prilocaine hydrochloride, and lidocaine hydrochloride; bronchodilators such as salbutamol hemisulfate, terbutaline sulfate, tulobuterol hydrochloride, isoproterenol hydrochloride, clorprenaline hydrochloride, orciprenaline sulfate, and methoxyphenamine hydrochloride; antitussive medicines such as alloclamide hydrochloride, oxeladin citrate, carbetapentane citrate, ephedrine hydrochloride, cloperastine hydrochloride, codeine phosphate, and dihydrocodeine phosphate; and antihistamines such as alimemazine tartrate, triprolidine hydrochloride, carbinoxamine meleate, chlorpheniramine maleate, diphenylpyraline hydrochloride, promethazine hydrochloride, and homochlorcyclizine hydrochloride.

The anionic water-soluble medicines usable in the present invention preferably include those having an anionic group such as a carboxyl group or a phosphate group, a pKa of 4.5 or less, and a solubility such that 1 g of the medicine can be dissolved in an amount of less that 10 ml of water at 25°C. Typical examples of such medicines are antiallergic medicines such as disodium cromoglycate and sodium tranilast; psychotropic medicines such as dipotassium clorazepate; and adrenocortical hormones such as dexamethasone sodium phosphate and betamethasone disodium phosphate.

The ionic oil-soluble substances and their salts usable as the accelerator according to the present invention include acidic oil-soluble substances and their salts having in the molecule thereof, an acidic group such as a carboxylic, sulfuric, sulfonic, or phosphoric acid group and basic oil-soluble substances and their salts having, in the molecule thereof, a basic group such as a primary, secondary, tertiary, or quaternary amino group.

Typical examples of the acidic oil-soluble substances and their salts usable in the present invention are cholic acid derivatives and their salts such as cholic acid, dehydrocholic acid, glycocholic acid, taurocholic acid, chenodeoxycholic acid, taurodeoxycholic acid, ursodeoxycholic acid, and their salts (e.g., sodium and potassium salts), preferably dehydrocholic acid, taurocholic acid, and their salts; ethacrynic acid and their salts (e.g., sodium and potassium salts); and anionic surfactants such as alkyl sulfates (e.g., alkyl sulfates having a $C_6$-$C_{20}$, preferably $C_{10}$-$C_{18}$ alkyl group and their salts (e.g., sodium and potassium salts), preferably sodium lauryl sulfate and sodium cetyl sulfate), polyoxyethylene alkyl ether sulfates and their salts (e.g., polyoxyethylene alkyl ether sulfates having a $C_6$-$C_{20}$, preferably $C_{10}$-$C_{18}$ alkyl group and a 1 to 20, preferably 2 to 12 mole number of added ethyleneoxide and their salts (e.g., sodium and potassium salts), preferably sodium polyoxyethylene lauryl ether sulfates and sodium polyoxyethylene cetyl ether sulfates), polyoxyethylene alkyl ether phosphates and their salts (e.g., polyoxyethylene alkyl ether mono-, di-, or triphosphates having a $C_6$-$C_{20}$, preferably $C_{10}$-$C_{18}$ alkyl group and a 1 to 20, preferably 2 to 10 mole number of added ethyleneoxide and their salts (e.g., sodium and potassium salts), preferably sodium polyoxyethylene lauryl ether phosphates, polyoxyethylene cetyl ether phosphates, sodium polyoxyethylene cetyl ether phosphates, polyoxyethylene oleyl ether phosphates, and sodium polyoxyethylene oleyl ether phosphates), and sarcosine derivatives and their salts (e.g., acid amides of fatty acids having a $C_6$-$C_{20}$, preferably $C_{10}$-$C_{18}$ alkyl group with N-methylglycine or N-methyl-β-alanine and their salts (e.g., sodium and potassium salts), preferably sodium lauroyl sarcosinate).

Typical examples of the basic oil-soluble substances and their salts usable in the present invention are alkanol amines and their salts such as the mono-, di-, and triamines having a $C_2$-$C_8$ alkanol group (e.g., monoethanolamine, diethanolamine, triethanolamine, and diisopropanolamine) and their salts (e.g., hydrochloride, sulfate, and phosphate), preferably diethanolamine and triethanolamine; alkyl diamines such as those having a $C_2$-$C_8$ alkyl group, preferably ethylenediamine and propylenediamine; and choline and its salts (e.g., chloride, bromide, and iodide), preferably choline chloride.

According to the present invention, the above-mentioned acidic oil-soluble substances and their salts are used as the absorption accelerator for the cationic water-soluble medicines. On the other hand, the above-mentioned basic oil-soluble substances and their salts are used as the absorption accelerator for the anionic water-soluble medicines.

The amphoteric surfactants usable as the accelerator according to the present invention include betaine type amphoteric surfactants such as alkyl betaines, especially having, on the nitrogen atom, a $C_1$-$C_{20}$ , preferably $C_{10}$-$C_{18}$ alkyl group, two methyl or hydroxyethyl groups, and an acetic or propionic acid group (e.g., lauryl dimethylamino acetic acid betaine, lauryl dimethylamino propionic acid betaine, myristyl dimethylamino acetic acid betaine, lauryl dimethylamino propionic acid betaine, myristyl dimethylamino acetic acid betaine, myristyl dimethylamino propionic acid betaine, stearyl dimethylamino acetic acid betaine, and stearyl dihydroxyethylamino acetic acid betaine, especially preferably lauryl dimethylamino acetic acid betaine and imidazolinium betaines such as 2-alkyl-N-carboxymethyl-N-hydroxyethyl imidazolinium betaines especially having a $C_1$-$C_{20}$ , preferalby $C_{10}$-$C_{18}$ alkyl group (e.g., 2-undecyl-N-carboxyl-methyl-N-hydroxyethyl imidazolinium betaine, 2-lauryl-N-carboxymethyl-N-hydroxyethyl imidazolinium betaine, 2-oleyl-N-carboxymethyl-N-hydroxyethyl imidazolinium betaine), especially 2-undecyl-N-carboxymethyl-N-hydroxyethyl imidazolinium betaine; amino acid type amphoteric surfactants such as alkylamino acids

having a $C_1$-$C_{20}$ , preferably $C_{10}$-$C_{18}$ alkyl group (e.g., N-lauryl-β-alanine, N-stearyl-β-alanine, and lauryl diaminoethyl glycine); sulfonic acid type amphoteric surfactants such as alkylamino sulfonic acids and alkyl imidazoline sulfonic acids both having a $C_6$-$C_{20}$ , preferably $C_{10}$-$C_{18}$ alkyl group (e.g., decyl methylaminopropyl sulfonic acid, lauryl dimethylaminopropyl sulfonic acid, palmityl dimethylpropyl sulfonic acid, lauryl di-n-propylaminopropyl sulfonic acid, and 2-undecyl-N-sulfonoethyl imidazoline, preferably lauryl dimethylaminopropyl sulfonic acid and 2-undecyl-N-sulfonoethyl imidazoline); and sulfuric acid ester (i.e., sulfate) type amphoteric surfactants such as alkylamino sulfates and 2-undecyl-N-sulfoethyl imidazolines both having a $C_6$-$C_{20}$ , preferably $C_{10}$-$C_{18}$ alkyl group (e.g., decyl dimethylaminopropyl sulfate, lauryl dimethylaminopropyl sulfate, lauryl di-n-propylaminopropyl sulfate, and 2-undecyl-N-sulfoethyl imidazoline, preferably lauryl dimethylaminopropyl sulfate and 2-undecyl-N-sulfoethyl imidazoline).

The nonionic substances usable as the accelerator according to the present invention include pantothenic acid derivatives, p-aminobenzoic acid derivatives, sucrose fatty acid esters, polyoxyethylene sorbitan fatty acid esters, and polyoxyethylene alkyl ethers.

Typical examples of the pantothenic acid derivatives are esters of pantothenic acid with a fatty acid having a $C_1$-$C_5$ alkyl group, preferably D-dicarbethoxy ethyl pantothenate.

Typical examples of the p-aminobenzoic acid derivatives are esters of a fatty acid having a $C_1$-$C_5$ alkyl group, preferably ethyl p-aminobenzoate and propyl p-aminobenzoate.

Typical examples of the sucrose fatty acid esters are mono-, di-, and triesters of sucrose with a fatty acid having a $C_2$-$C_{18}$ , preferably $C_{10}$-$C_{18}$ alkyl group, preferably sucrose monolaurate, sucrose dimyristate, sucrose monopalmitate, sucrose trilaurate, sucrose distearate, sucrose monooleate, and sucrose tallow esters.

Typical examples of polyoxyethylene sorbitan fatty acid esters are mono- and triesters of fatty acids having a $C_4$-$C_{20}$ , preferably $C_{10}$-$C_{18}$ alkyl group with a 1 to 50 mole, preferably 2 to 23 mole number of added ethyleneoxide such as polyoxyethylene sorbitan monolaulate, polyoxyethylene sorbitan monopalmitate, polyoxyethylene sorbitan monostearate, polyoxyethylene sorbitan tristearate, polyoxyethylene sorbitan monooleate, polyoxyethylene sorbitan trioleate, and polyoxyethylene sorbitan monoisostearate. The preferable polyoxyethylene sorbitan fatty acid ester is polyoxyethylene sorbitan monooleate.

Typical examples of polyoxyethylene alkyl ethers are those having a $C_4$-$C_{20}$ , preferably $C_{10}$-$C_{18}$ alkyl group and a 1 to 50 mole, preferably 2 to 23 mole number of added ethylenoxide such as polyoxyethylene lauryl ether, polyoxyethylene cetyl ether, polyoxyethylene stearyl ether, and polyoxyethylene oleyl ether. The preferable polyoxyethylene alkyl ethers are polyoxyethylene lauryl ether and polyoxyethylene cetyl ether.

The above-mentioned percutaneous absorption accelerators including ionic oil-soluble substances and their salts, amphoteric surfactants, and nonionic substances may be used alone or in any mixture thereof.

Although there are no critical limitations to the amount of the percutaneous absorption accelerator according to the present invention, the percutaneous absorption accelerator may be usually used in an amount of 2 to 6000 parts by weight, preferably 5 to 1500 parts by weight, based on 100 parts by weight of the ionic water-soluble medicines, depending upon, for example, the types of the medicines. The use of a too small amount of the accelerator tends to result in insufficient absorption acceleration effects. Contrary to this, the use of a too large amount of the accelerator tends to result in an unpreferable skin irritation.

As mentioned above, the desired percutaneous absorption acceleration of cationic or anionic water-soluble medicines can be obtained by the use of one component of the above-mentioned ionic oil-soluble substances and their salts, amphoteric surfactants, and nonionic substances. However, when at least one acidic oil-soluble substance or its salt or at least one amphoteric surfactant is used in combination with at least one nonionic substance, the percutaneous absorption of the cationic water-soluble medicines can be synergistically accelerated. Similarly, when at least one basic oil-soluble substance or its salt or at least one amphoteric surfactant is used in combination with at least one nonionic substance, the percutaneous absorption of the anionic water-soluble medicines can be synergistically accelerated.

The percutaneous absorption accelerator according to the present invention can be advantageoulsy incorporated into transdermal therapeutic preparations, together with ionic water-soluble medicines. The transdermal therapeutic preparations can be in any conventional shape, for example, ointments, gels, poultices, films.

The water-soluble polymeric substances usable as a base in the transdermal therapeutic preparations includes those conventionally used as a base component in, for example, ointments, gels, poultices, and films. Typical examples of such polymer bases are alginic acid, sodium alginate, polyethylene glycol, methylcellulose, ethylcellulose, hydroxyethylcellulose, gelatin, polyvinyl alcohol, polyvinyl pyrrolidone, polyethyleneoxide, polyacrylic acid, sodium polyacrylate, and the crosslinked products thereof.

The transdermal therapeutic preparations according to the present invention can optionally contain any conventional ingredients such as emulsifiers (e.g., lecithine, gum arabic), humectants (e.g. propylene glycol, glycerol, sorbitol), and other conventional additivies.

When the transdermal therapeutic preparations are formulated, the ionic water-soluble medicines are generally used in an amount of 0.3% to 45% by weight, although the amount may be varied depending upon the types of medicines and the shapes of preparations. However, the use of a too small amount of the medicine tends to result in the insufficient percentaneous absorption amount of the medicine, whereas the use of a too large amount of the medicine tends to cause the precipitation of the medicine in the base or the decrease in the shaping ability of the base. Similarly, the use of a too small amount of the polymer base tends to result in the poor shaping ability of the preparations. Contrary to this, when a too large amount of the polymer base is used, the desired preparations having a sufficient amount of the medicine cannot be obtained. As mentioned above, the use of a too small amount of the percutaneous absorption accelerator results in the insufficicinet percutaneous absorption acceleration effects, whereas the use of a too large amount of the percutaneous absorption accelerator tends to cause unpreferable skin irritation.

When the oil-soluble substance or the amphoteric surfactant is combined with the nonionic substance, they can be preferably combined in a ratio of 2:8 to 8:2, more preferably 3:7 to 7:3 to obtain the desired synergetic percutaneous absorption acceleration effects.

As mentioned above, the transdermal therepeutic preparations according to the present invention can be formed in any conventional shapes including ointments, gels, poultices, and films. For example, when the preparations in the form of films and gels are produced, the above-mentioned water-soluble polymer substances and humectants (e.g., glycerol) are dissolved or suspended in water to form sols and the ionic water-soluble medicines and the percutaneous absorption accelerator are dissolved or suspended in the resultant sols to form gels. The desired preparations in the form of a film can be produced from the resultant gels in any conventional manner.

When the ointments are produced, the ionic water-soluble medicines and the percutaneous abosrption accelerators are suspended in a small amount of water and the resultant suspension is gradually mixed with the water-soluble polymeric substances. The desired ointments can be produced from the resultant mixture in any conventional manner.

Furthermore, when the preparations in the form of poultices are produced, mixtures of the water-soluble polymeric substances, excipients, humectants, and water are mixed with the ionic water-soluble medicines and the percutaneous absorption accelerators. The desired poultices can be produced from the resultant mixtures in any conventional manner.

The resultant transdermal therapeutic preparations can be percutaneously or transdermally applied to the human bodies in any conventional manner depending upon the intended use.

EXAMPLES

The present invention will now be further illustrated in detail by, but is by no means limited to, the following Examples.

Example 1

A 0.6 g amount of polyvinyl alcohol (GM 1400 available from IWAI KAGAKU YAKUHIN K.K., Japan) was gradually added to 7 ml of purified water, while stirring, at room temperature to dissolve the polyvinyl alcohol in the water. Then, 0.6 g of glycerol was added to the resultant solution, while stirring, at room temperature. Thus, a sol product was prepared.

To the resultant sol product, 0.6 g of diltiazem hydrochloride and 0.1 g of dehydrocholic acid were added to prepare a gel product.

The gel product obtained above was spread or coated onto a polyethylene film support having a size of about 16 x 16 cm square, followed by heating at a temperature of about 50°C for 15 hours.

As a result, the dried transdermal therapeutic preparations in the form of a film were obtained.

Example 2

The transdermal therapeutic preparations in the form of a film were obtained in the same manner as in Example 1, except that 0.1 g of D-dicarbethoxy ethyl pantothenate was used instead of the dehydrocholic acid.

Example 3

The transdermal therapeutic preparations in the form of a film were obtained in the same manner as in Example 1, except that 0.1 g of ethacrynic acid was used instead of the dehydrocholic acid.

Example 4

The transdermal therapeutic preparations in the form of a film were obtained in the same manner as in Example 1, except that 0.1 g of polyoxyethylene (20) sorbitane monooleate was used instead of the dehydrocholic acid.

Example 5

The transdermal therapeutic preparations in the form of a film were obtained in the same manner as in Example 1, except that 0.1 g of sucrose fatty acid ester (Ryoto Sugar Esters S 1670 available from MITSUBISHI CHEMICAL IND., CO.) was used instead of the dehydrocholic acid.

Example 6

The transdermal therapeutic preparations in the form of a film were obtained in the same manner as in Example 1, except that 0.1 g of polyoxyethylene (9) lauryl ether was used instead of the dehydrocholic acid.

Example 7

The transdermal therapeutic preparations in the form of a film were obtained in the same manner as in Example 1, except that 0.1 g of sodium polyoxyethylene (4) lauryl etehr sulfate was used instead of the dehydrocholic acid.

Example 8

The transdermal therapeutic preparations in the form of a film were obtained in the same manner as in Example 1, except that 0.1 g of sodium polyoxyethylene (4) lauryl ether phosphate was used instead of the dehydrocholic acid.

Example 9

The transdermal therapeutic preparations in the form of a film were obtained in the same manner as in Example 1, except that 0.1 g of sodium lauryl sulfate was used instead of the dehydrocholic acid.

Example 10

The transdermal therapeutic preparations in the form of a film were obtained in the same manner as in Example 1, except that 0.1 g of sodium lauroyl sarcosinate was used instead of the dehydrocholic acid.

Example 11

The transdermal therapeutic preparations in the form of a film were obtained in the same manner as in Example 1, except that 0.1 g of lauryl dimethylamino acetic acid betaine was used instead of the dehydrocholic acid.

Example 12

The transdermal therapeutic preparations in the form of a film were obtained in the same manner as in Example 1, except that 0.1 g of 2-undecyl-N-carboxymethyl-N-hydroxyethyl imidazolinium betaine was used instead of the dehydrocholic acid.

Example 13

The transdermal therapeutic preparations in the form of a film were obtained in the same manner as in Example 1, except that 0.1 g of lauryl diaminoethyl glycine was used instead of the dehydrocholic acid.

Example 14

The transdermal therapeutic preparations in the form of a film were obtained in the same manner as in Example 1, except that 0.1 g of 2-undecyl-N-sulfoethyl imidazoline was used instead of the dehydrocholic acid.

Example 15

The transdermal therapeutic preparations in the form of a film were obtained in the same manner as in Example 1, except that 0.05 g of sodium lauryl sulfate and 0.05 g of polyoxyethylene (9) lauryl ether were used instead of the dehydrocholic acid.

Example 16

The transdermal therapeutic preparations in the form of a film were obtained in the same manner as in Example 15, except that 0.06 g D-dicarbethoxyethyl pantothenate was used instead of the polyoxyethylene (9) lauryl ether.

Example 17

The transdermal therapeutic preparations in the form of a film were obtained in the same manner as in Example 15, except that 0.09 of sucrose fatty acid ester (see Example 5) was used instead of the polyoxyethylene (9) lauryl ether.

Example 18

The transdermal therapeutic preparations in the form of a film were obtained in the same manner as in Example 15, except that 0.05 g of sodium polyoxyethylene (4) lauryl ether, phosphate was used instead of the sodium lauryl sulfate.

Example 19

The transdermal therapeutic preparations in the form of a film were obtained in the same manner as in Example 18, except that 0.06 g of D-dicarbethoxy ethyl pantothenate was used instead of the polyoxyethylene (9) lauryl ether.

Example 20

The transdermal therapeutic preparations in the form of a film were obtained in the same manner as in Example 18, except that 0.09 g of sucrose fatty acid ester (see Example 5) was used instead of the polyoxyethylene (9) lauryl ether.

Example 21

The transdermal theapeutic preparations in the form of a film were obtained in the same manner as in Example 15, except that 0.05 g of sodium polyoxyethylene (4) lauryl ether sulfate was used instead of the sodium lauryl sulfate.

Example 22

The transdermal therapeutic preparations in the form of a film were obtained in the same manner as in Example 21, except that 0.06 g of D-dicarbethoxy ethyl pantothenate was used instead of the polyoxyethylene (9) lauryl ether.

Example 23

The transdermal therapeutic preparations in the form of a film were obtained in the same manner as in Example 21, except that 0.09 g of sucrose fatty acid ester (see Example 5) was used instead of the polyoxyethylene (9) lauryl ether.

Example 24

The transdermal therapeutic preparations in the form of a film were obtained in the same manner as in Example 15, except that 0.05 g of sodium lauroyl sarcosinate was used instead of the sodium lauryl sulfate.

Example 25

The transdermal therapeutic preparations in the form of a film were obtained in the same manner as in Example 24, except that 0.06 g of D-dicarbethoxy ethyl pantothenate was used instead of the polyoxyethylene (9) lauryl ether.

Example 26

The transdermal therapeutic preparations in the form of a film were obtained in the same manner as in Example 15, except that 0.05 g of 2-undecyl-N-carboxymethyl-N-hydroxyethyl imidazolinium betaine was used instead of the sodium lauryl sulfate.

Example 27

The transdermal therapeutic preparations in the form of a film were obtained in the same manner as in Example 26, except that 0.06 g of D-dicarbethoxy ethyl pantothenate was used instead of the polyoxyethylene (9) lauryl ether.

Example 28

The transdermal therapeutic preparations in the form of a film were obtained in the same manner as in Example 26, except that 0.09 g of sucrose fatty acid ester (see Example 5) was used instead of the polyoxyethylene (9) lauryl ether.

Example 29

The transdermal therapeutic preparations in the form of a film were obtained in the same manner as in Example 15, except that 0.05 g of lauryl dimethylamino acetic acid betaine was used instead of the sodium lauryl sulfate.

Example 30

The transdermal therapeutic preparations in the form of a film were obtained in the same manner as in Example 8, except that 0.2 g of acebutolol hydrochloride was used instead of the diltiazem hydrochloride.

Example 31

The transdermal therapeutic preparations in the form of a film were obtained in the same manner as in Example 30, except that 0.1 g of sodium polyoxyethylene (4) lauryl ether sulfate was used instead of the sodium polyoxyethylene (4) lauryl ether phosphate.

Example 32

The transdermal therapeutic preparations in the form of a film were obtained in the same manner as in Example 30, except that 0.1 g of polyoxyethylene (9) lauryl ether was used instead of the sodium polyoxyethylene (4) lauryl ether phosphate.

Example 33

The transdermal therapeutic preparations in the form of a film were obtained in the same manner as in Example 30, except that the amount of the sodium polyoxyethylene (4) lauryl ether phosphate was changed to 0.05 g and 0.05 g of polyoxyethylene (9) lauryl ether was further used.

Example 34

The transdermal therapeutic preparations in the form of a film were obtained in the same manner as in Example 31, except that the amount of the sodium polyoxyethylene (4) lauryl ether sulfate was changed to 0.05 g and 0.05 g of polyoxyethylene (9) lauryl ether was further used.

Example 35

The transdermal therapeutic preparations in the form of a film were obtained in the same manner as in Example 31, except that 0.3 g of alprenolol hydrochloride was used instead of the acebutolol hydrochloride.

Example 36

The transdermal therapeutic preparations in the form of a film were obtained in the same manner as in Example 35, except that 0.1 g of polyoxyethylene (9) lauryl ether was used instead of the sodium polyoxyethylene (4) lauryl ether sulfate.

Example 37

The transdermal therapeutic preparations in the form of a film were obtained in the same manner as in Example 35, except that the amount of the sodium polyoxyethylene (4) lauryl ether sulfate was changed to 0.05 g and 0.05 g of polyoxyethylene (9) lauryl ether was further used.

Example 38

The transdermal therapeutic preparations in the form of a film were obtained in the same manner as in Example 12, except that 0.4 g of metoprolol tartrate was used instead of the diltiazem hydrochloride.

Example 39

The transdermal therapeutic preparations in the form of a film were obtained in the same manner as in Example 38, except that 0.1 g of polyoxyethylene (9) lauryl ether was used instead of the 2-undecyl-N-carboxymethyl-N-hydroxyethyl imidazolinium betaine.

Example 40

The transdermal therapeutic preparations in the form of a film were obtained in the same manner as in Example 38, except that the amount of 2-undecyl-N-carboxymethyl-N-hydroxyethyl imidazolinium betaine was changed to 0.05 g and 0.05 g of polyoxyethylene (9) lauryl ether was further used.

Example 41

The transdermal therapeutic preparations in the form of a film were obtained in the same manner as in Example 10, except that 0.1 g of trimetazidine hydrochloride was used instead of the diltiazem hydrochloride.

Example 42

The transdermal therapeutic preparations in the form of a film were obtained in the same manner as in Example 41, except that the amount of sodium lauroyl sarcosinate was changed to 0.05 g and 0.09 g of sucrose fatty acid ester (see Example 5) was further used.

Example 43

The transdermal therapeutic preparations in the form of a film were obtained in the same manner as in Example 7, except that 0.6 g of disopyramide phosphate was used instead of the diltiazem hydrochloride.

Example 44

The transdermal therapeutic preparations in the form of a film were obtained in the same manner as in Example 43, except that 0.1 g of sucrose fatty acid ester (see Example 5) was used instead of the sodium polyoxyethylene (4) lauryl ether sulfate.

Example 45

The transdermal therapeutic preparations in the form of a film were obtained in the same manner as in Example 43, except that the amount of sodium polyoxyethylene (4) lauryl ether sulfate was changed to 0.05 g and 0.09 g of sucrose fatty acid ester was further used.

Example 46

The transdermal therapeutic preparations in the form of a film were obtained in the same manner as in Example 33, except that 0.6 g of octopamine hydrochloride was used instead of the acebutolol hydrochloride.

Example 47

The transdermal therapeutic preparations in the form of a film were obtained in the same manner as in Example 2, except that 0.9 g of imipramine hydrochloride was used instead of the diltiazem hydrochloride.

Example 48

The transdermal therapeutic preparations in the form of a film were obtained in the same manner as in Example 47, except that 0.1 g of polyoxyethylene (9) lauryl ether was used instead of the D-dicarbetoxy ethyl pantothenate.

Example 49

The transdermal therapeutic preparations in the form of a film were obtained in the same manner as in Example 4, except that 0.3 g of chlorpromazine hydrochloride was used instead of the diltiazem hydrochloride.

Example 50

The transdermal therapeutic preparations in the form of a film were obtained in the same manner as in Example 49, except that the amount of the polyoxyethylene (20) sorbitan monooleate was changed to

0.05 g and 0.05 g of dimethylamino acetic acid betaine was further used.

Example 51

The transdermal therapeutic preparations in the form of a film were obtained in the same manner as in Example 8, except that 0.2 g of benzydamine hydrochloride was used instead of the diltiazem hydrochloride.

Example 52

The transdermal therapeutic preparations in the form of a film were obtained in the same manner as in Example 51, except that the amount of sodium polyoxyethylene (4) lauryl ether phosphate was changed to 0.05 g and 0.09 g of sucrose fatty acid ester (see Example 5) was further used.

Example 53

The transdermal therapeutic preparations in the form of a film were obtained in the same manner as in Example 7, except that 0.3 g of scopolamine butylbromide was used instead of the diltiazem hydrochloride.

Example 54

The transdermal therapeutic preparations in the form of a film were obtained in the same manner as in Example 53, except that 0.1 g of sodium polyoxyethylene (4) lauryl ether phosphate was used instead of the sodium polyoxyethylene (4) lauryl ether sulfate.

Example 55

The transdermal therapeutic preparations in the form of a film were obtained in the same manner as in Example 53, except that the amount of sodium polyoxyethylene (4) lauryl ether sulfate was changed to 0.05 g and 0.05 g of polyoxyethylene (9) lauryl ether was further used.

Example 56

The transdermal therapeutic preparations in the form of a film were obtained in the same manner as in Example 54, except that the amount of sodium polyoxyethylene (4) lauryl ether phosphate was changed to 0.05 g and 0.09 g of sucrose fatty acid ester (see Example 5) was further used.

Example 57

The transdermal therapeutic preparations in the form of a film were obtained in the same manner as in Example 8, except that 0.5 g of ketamine hydrochloride was used instead of the diltiazem hydrochloride.

Example 58

The transdermal therapeutic preparations in the form of a film were obtained in the same manner as in Example 57, except that 0.1 g of polyoxyethylene (9) lauryl ether was used instead of the sodium polyoxyethylene (4) lauryl ether phosphate.

Example 59

The transdermal therapeutic preparations in the form of a film were obtained in the same manner as in Example 57, except that 0.1 g of sodium polyoxyethylene (4) lauryl ether phosphate and 0.1 g of polyoxyethylene (9) lauryl ether were used.

Example 60

The transdermal therapeutic preparations in the form of a film were obtained in the same manner as in Example 12, except that 0.6 g of lidocaine hydrochloride was used instead of the diltiazem hydrochloride.

Example 61

The transdermal therapeutic preparations in the form of a film were obtained in the same manner as in Example 60, except that 0.1 g of polyoxyethylene (9) lauryl ether was used instead of the 2-undecyl-N-carboxymethyl-N-hydroxyethyl imidazolinium betaine.

Example 62

The transdermal therapeutic preparations in the form of a film were obtained in the same manner as in Example 60, except that the amount of 2-undecyl-N-carboxymethyl-N-hydroxyethyl imidazolinium betaine was changed to 0.05 g and 0.05 g of polyoxyethylene (9) lauryl ether was further used.

Example 63

The transdermal therapeutic preparations in the form of a film were obtained in the same manner as in Example 7, except that 0.055 g of salbutamol hemisulfate was used instead of the diltiazem hydrochloride.

Example 64

The transdermal therapeutic preparations in the form of a film were obtained in the same manner as in Example 63, except that 0.1 g of sodium polyoxyethylene (4) lauryl ether phosphate was used instead of the sodium polyoxyethylene (4) lauryl ether sulfate.

Example 65

The transdermal therapeutic preparations in the form of a film were obtained in the same manner as in Example 63, except that 0.1 g of 2-undecyl-N-carboxymethyl-N-hydroxyethyl imidazolinium betaine was used instead of the sodium polyoxyethylene (4) lauryl ether sulfate.

Example 66

The transdermal therapeutic preparations in the form of a film were obtained in the same manner as in Example 63, except that 0.1 g of polyoxyethylene (9) lauryl ether was used instead of the sodium polyoxyethylene (4) lauryl ether sulfate.

Example 67

The transdermal therapeutic preparations in the form of a film were obtained in the same manner as in Example 63, except that 0.1 g of sodium lauroyl sarcosinate was used instead of the sodium polyoxyethylene (4) lauryl ether sulfate.

Example 68

The transdermal therapeutic preparations in the form of a film were obtained in the same manner as in Example 63, except that 0.1 g of lauryl dimethylamino acetic acid betaine was used instead of the sodium polyoxyethylene (4) lauryl ether sulfate.

Example 69

The transdermal therapeutic preparations in the form of a film were obtained in the same manner as in Example 63, except that 0.1 g of sodium lauryl sulfate was used instead of the sodium polyoxyethylene (4) lauryl ether sulfate.

Example 70

The transdremal therapeutic preparations in the form of a film were obtained in the same manner as in Example 63, except that 0.1 g of sucrose fatty acid ester (see Example 5) was used instead of the sodium polyoxyethylene (4) lauryl ether sulfate.

Example 71

The transdermal therapeutic preparations in the form of a film were obtained in the same manner as in Example 63, except that 0.1 g of D-dicarbethoxy ethyl pantothenate was used instead of the sodium polyoxyethylene (4) lauryl ether sulfate.

Example 72

The transdermal therapeutic preparations in the form of a film were obtained in the same manner as in Example 63, except that the amount of the sodium polyoxyethylene (4) lauryl ether sulfate was changed to 0.05 g and 0.05 g of polyoxyethylene (9) lauryl ether was further used.

Example 73

The transdermal therapeutic preparations in the form of a film were obtained in the same manner as in Example 64, except that the amount of sodium polyoxyethylene (4) lauryl ether phosphate was changed to 0.05 g and 0.05 g of polyoxyethylene (9) lauryl ether was further used.

Example 74

The transdermal therapeutic preparations in the form of a film were obtained in the same manner as in Example 65, except that the amount of 2-undecyl-N-carboxymethyl-N-hydroxyethyl imidazolinium betaine was changed to 0.05 g and 0.05 g of polyoxyethylene (9) lauryl ether was further used.

Example 75

The transdermal therapeutic preparations in the form of a film were obtained in the same manner as in Example 73, except that 0.09 g of sucrose fatty acid ester (see Example 5) was used instead of the polyoxyethylene (9) lauryl ether.

Example 76

The transdermal therapeutic preparations in the form of a film were obtained in the same manner as in Example 63, except that 0.055 g of terbutaline sulfate was used instead of the salbutamol hemisulfate.

Example 77

The transdermal therapeutic preparations in the form of a film were obtained in the same manner as in Example 76, except that 0.1 g of 2-undecyl-N-carboxymethyl-N-hydroxyethyl imidazolinium betaine was used instead of the sodium polyoxyethylene (4) lauryl ether sulfate.

Example 78

The transdermal therapeutic preparations in the form of a film were obtained in the same manner as in Example 76, except that 0.1 g of polyoxyethylene (9) lauryl ether was used instead of the sodium polyoxyethylene (4) lauryl ether sulfate.

Example 79

The transdermal therapeutic preparations in the form of a film were obtained in the same manner as in Example 76, except that the amount of sodium polyoxyethylene (4) lauryl ether sulfate was changed to 0.05 g and 0.05 g of polyoxyethylene (9) lauryl ether was further used.

Example 80

The transdermal therapeutic preparations in the form of a film were obtained in the same manner as in Example 77, except that the amount of 2-undecyl-N-carboxymethyl-N-hydroxyethyl imidazolinium betaine was changed to 0.05 g and 0.55 g of polyoxyethylene (9) lauryl ether was further used.

Example 81

The transdermal therapeutic preparations in the form of a film were obtained in the same manner as in Example 63, except that 0.01 g of tulobuterol hydrochloride was used instead of the salbutamol hemisulfate.

Example 82

The transdermal therapeutic preparations in the form of a film were obtained in the same manner as in Example 81, except that 0.1 g of 2-undecyl-N-carboxymethyl-N-hydroxyethyl imidazolinium betaine was used instead of the sodium polyoxyethylene (4) lauryl ether sulfate.

Example 83

The transdermal therapeutic preparations in the form of a film were obtained in the same manner as in Example 81, except that 0.1 g of polyoxyethylene (9) lauryl ether was used instead of the sodium polyoxyethylene (4) lauryl ether sulfate.

Example 84

The transdermal therapeutic preparations in the form of a film were obtained in the same manner as in Example 81, except that the amount of sodium polyoxyethylene (4) lauryl ether sulfate was changed to 0.05 g and 0.05 g of polyoxyethylene (9) lauryl ether was further used.

Example 85

The transdremal therapeutic preparations in the form of a film were obtained in the same manner as in Example 8, except that 0.6 g of alloclamide hydrochloride was used instead of the diltiazem hydrochloride.

Example 86

The transdermal therapeutic preparations in the form of a film were obtained in the same manner as in Example 85, except that 0.1 g of lauryl dimethylamino acetic acid betaine was used instead of the sodium polyoxyethylene (4) lauryl ether phosphate.

Example 87

The transdermal therapeutic preparations in the form of a film were obtained in the same manner as in Example 7, except that 0.1 g of carbinoxamine maleate was used instead of the diltiazem hydrochloride.

Example 88

The transdermal therapeutic preparations in the form of a film were obtained in the same manner as in Example 87, except that the amount of the sodium polyoxyethylene (4) lauryl ether sulfate was changed to 0.05 g and 0.05 g of sucrose fatty acid ester (see Example 5) was further used.

Example 89

The transdermal therapeutic preparations in the form of a film were obtained in the same manner as in Example 8, except that 0.1 g of diphenylpyraline hydrochloride was used instead of the diltiazem hydrochloride.

Example 90

The transdermal therapeutic preparations in the form of a film were obtained in the same manner as in Example 1, except that 0.5 g of disodium cromoglycate was used instead of the diltiazem hydrochloride and 0.1 g of triethanolamine was used instead of the dehydrocholic acid.

Example 91

The transdermal therapeutic preparations in the form of a film were obtained in the same manner as in Example 90, except that 0.1 g of triethanolamine lauryl sulfate was used instead of the triethanolamine.

Example 92

The transdermal therapeutic preparations in the form of a film were obtained in the same manner as in Example 90, except that 0.1 g of ethylene diamine was used instead of the triethanolamine.

Example 93

The transdermal therapeutic preparations in the form of a film were obtained in the same manner as in Example 90, except that 0.1 g of lauryl dimethylamino acetic acid betaine was used instead of the triethanolamine.

Example 94

The transdermal therapeutic preparations in the form of a film were obtained in the same manner as in Example 90, except that 0.1 g of 2-undecyl-N-carboxymethyl-N-hydroxyethyl imidazolinium betaine was used instead of the triethanolamine.

Example 95

The transdermal therapeutic preparations in the form of a film were obtained in the same manner as in Example 90, except that 0.1 g of lauryl diaminoethyl glycine was used instead of the triethanolamine.

Example 96

The transdermal therapeutic preparations in the form of a film were obtained in the same manner as in Example 90, except that 0.1 g of 2-undecyl-N-sulfoethyl imidazoline was used instead of the triethanolamine.

Example 97

The transdermal therapeutic preparations in the form of a film were obtained in the same manner as in Example 90, except that 0.1 g of polyoxyethylene (9) lauryl ether was used instead of the triethanolamine.

Example 98

The transdermal therapeutic preparations in the form of a film were obtained in the same manner as in Example 90, except that 0.1 g of sucrose fatty acid ester (see Example 5) was used instead of the triethanolamine.

Example 99

The transdermal therapeutic preparations in the form of a film were obtained in the same manner as in Example 90, except that 0.1 g of D-dicarbethoxy ethyl pantothenate was used instead of the triethanolamine.

Example 100

The transdermal therapeutic preparations in the form of a film were obtained in the same manner as in Example 90, except that 0.1 g of choline chloride was used instead of the triethanolamine.

Example 101

The transdermal therapeutic preparations in the form of a film were obtained in the same manner as in Example 90, except that the amount of triethanolamine was changed to 0.05 g and 0.05 g of polyoxyethylene (9) lauryl ether was further used.

Example 102

The transdermal therapeutic preparations in the form of a film were obtained in the same manner as in Example 100, except that the amount of choline chloride was changed to 0.05 g and 0.05 g of polyoxyethylene (9) lauryl ether was further used.

Example 103

The transdermal therpeutic preparations in the form of a film were obtained in the same manner as in Example 102, except that 0.06 g of D-dicarbethoxy ethyl pantothenate was used instead of the polyoxyethylene (9) lauryl ether.

Example 104

The transdermal therapeutic preparations in the form of a film were obtained in the same manner as in Example 102, except that 0.09 g of sucrose fatty acid ester (see Example 5) was used instead of the polyoxyethylene (9) lauryl ether.

Example 105

The transdermal therapeutic preparations in the form of a film were obtained in the same manner as in Example 97, except that 0.1 g of dipotassium cloazepate was used instead of the disodium cromoglycate.

Example 106

The transdermal therapeutic preparations in the form of a film were obtained in the same manner as in Example 105, except that 0.1 g of choline chloride was used instead of the polyoxyethylene (9) lauryl ether.

Example 107

The transdermal therapeutic preparations in the form of a film were obtained in the same manner as in Example 94, except that 0.5 g of dexamethasone sodium phosphate was used instead of the disodium cromoglycate.

Example 108

A 0.3 g amount of polyvinyl alcohol (GM 1400 available from IWAI KAGAKU YAKUHIN K.K., Japan) was gradually added to 7 ml of purified water at room temperature, while stirring. To the resultant solution, 0.3 g of glycerol was added, while stirring, to form a sol.

To the resultant sol, 0.6 g of diltiazem hydrochloride, 0.1 g of sodium polyoxyethylene (4) lauryl ether phosphate, and 0.1 g of polyoxyethylene (9) lauryl ether were added and the mixture was heated at a temperature of 60°C, while stirring, to vaporize the water. Thus, 4 g of the desired transdermal therapeutic preparation in the form of a gel was obtained.

Example 109

The transdermal therapeutic preparations in the form of a gel were obtained in the same manner as in Example 108, except that 0.1 g of sodium polyoxyethylene (4) lauryl ether sulfate was used instead of the sodium polyoxyethylene (4) lauryl ether phosphate.

Example 110

The transdermal therapeutic preparations in the form of a gel were obtained in the same manner as in Example 108, except that 0.1 g of sucrose fatty acid ester (see Example 5) was used instead of the polyoxyethylene (9) lauryl ether.

Example 111

The transdermal therapeutic preparations in the form of a gel were obtained in the same manner as in Exmple 110, except that 0.1 g of 2-undecyl-N-carboxymethyl-N-hydroxyethyl imidazolinium betaine was used instead of the sodium polyoxyethylene (4) lauryl ether phosphate.

Example 112

The transdermal therapeutic preparations in the form of a gel were obtained in the same manner as in Example 108, except that 0.2 g of benzydamine hydrochloride was used instead of the diltiazem hydrochloride.

Example 113

The transdermal therapeutic preparations in the form of a gel were obtained in the same manner as in Example 112, except that 0.1 g of sucrose fatty acid ester (see Example 5) was used instead of the polyoxyethylene (9) lauryl ether.

Example 114

The transdermal therapeutic preparations in the form of a gel were obtained in the same manner as in Example 108, except that 0.5 g of ketamine hydrochloride was used instead of the diltiazem hydrochloride.

Example 115

The transdermal therapeutic preparations in the form of a gel were obtained in the same manner as in Example 108, except that 0.055 g of salbutamol hemisulfate was used instead of the diltiazem hydrochloride.

Example 116

The transdermal therapeutic preparations in the form of a gel were obtained in the same manner as in Example 115, except that 0.1 g of sodium polyoxyethylene (4) lauryl ether sulfate was used instead of the sodium polyoxyethylene (4) lauryl ether phosphate.

Example 117

The transdermal therapeutic preparations in the form of a gel were obtained in the same manner as in Example 115, except that 0.1 g of 2-undecyl-N-carboxymethyl-N-hydroxyethyl imidazolinium betaine was used instead of the sodium polyoxyethylene (4) lauryl ether phosphate.

Example 118

The transdermal therapeutic prepartions in the form of a gel were obtained in the same manner as in Example 116, except that 0.1 g of sucrose fatty acid ester (see Example 5) was used instead of the polyoxyethylene (9) lauryl ether.

Example 119

The transdermal therapeutic preparations in the form of a gel were obtained in the same manner as in Example 117, except that 0.1 g of sucrose fatty acid ester (see Example 5) was used instead of the polyoxyethylene (9) lauryl ether.

Example 120

The transdermal therapeutic preparations in the form of a gel were obtained in the same manner as in Example 115, except that 0.055 g of terbutaline sulfate was used instead of the salbutamol hemisulfate.

Example 121

The transdermal therapeutic preparations in the form of a gel were obtained in the same manner as in Example 120, except that 0.1 g of sodium polyoxyethylene (4) lauryl ether sulfate was used instead of sodium polyoxyethylene (4) lauryl ether phosphate.

Example 122

The transdermal therapeutic preparations in the form of a gel were obtained in the same manner as in Example 120, except that 0.1 g of 2-undecyl-N-carboxymethyl-N-hydroxyethyl imidazolinium betaine was used instead of the sodium polyoxyethylene (4) lauryl ether phosphate.

Example 123

The transdermal therapeutic preparations in the form of a gel were obtained in the same manner as in Example 120, except that 0.1 g of sodium lauryl sulfate was used instead of the sodium polyoxyethylene (4) lauryl ether phosphate.

Example 124

The transdermal therapeutic preparations in the form of a gel were obtained in the same manner as in Example 115, except that 0.01 g of tulobutenol hydrochloride was used instead of the salbutamol hemisulfate.

Example 125

The transdermal therapeutic preparations in the form of a gel were obtained in the same manner as in Example 24, except that 0.1 g of sodium polyoxyethylene (4) lauryl ether sulfate was used instead of the sodium polyoxyethylene (4) lauryl ether phosphate.

Example 126

The transdermal therapeutic preparations in the form of a gel were obtained in the same manner as in Example 124, except that 0.1 g of 2-undecyl-N-carboxymethyl-N-hydroxyethyl imidazolinium betaine was used instead of the sodium polyoxyethylene (4) lauryl ether phosphate.

Example 127

The transdermal therapeutic preparations in the form of a gel were obtained in the same manner as in Example 108, except that 0.1 g of carbinoxamine maleate was used instead of the diltiazem hydrochloride.

Example 128

The transdermal therapeutic preparations in the form of a gel were obtained in the same manner as in Example 127, except that 0.1 g of sucrose fatty acid ester (see Example 5) was used instead of the polyoxyethylene (9) lauryl ether.

Example 129

The transdermal therapeutic preparations in the form of a gel were obtained in the same manner as in Example 108, except that 0.5 g of disodium cromoglycate was used instead of the diltiazem hydrochloride and 0.1 g of triethanolamine was used instead of the sodium polyoxyethylene (4) lauryl ether phosphate.

Example 130

The transdermal therapeutic preparations in the form of a gel were obtained in the same manner as in Example 129, except that 0.1 g of ethylenediamine was used instead of the triethanolamine.

Example 131

The transdermal therapeutic preparations in the form of a gel were obtained in the same manner as in Example 129, except that 0.1 g of choline chloride was used instead of the triethanolamine.

Example 132

The transdermal therapeutic preparations in the form of a gel were obtained in the same manner as in Example 129, except that 0.1 g of 2-undecyl-N-carboxymethyl-N-hydroxyethyl amino acetic acid betaine was used instead of the triethanolamine.

Example 133

The transdermal therapeutic preparations in the form of a gel were obtained in the same manner as in Example 129, except that 0.1 g of sucrose fatty acid ester (see Example 5) was used instead of the polyoxyethylene (9) lauryl ether.

Example 134

The transdermal therapeutic preparations in the form of a gel were obtained in the same manner as in Example 130, except that 0.1 g of D-dicarbetoxy ethyl pantothenate was used instead of the polyoxyethylene (9) lauryl ether.

Example 135

The transdermal therapeutic preparations in the form of a gel were obtained in the same manner as in Example 131, except that 0.1 g of D-dicarbethoxy ethyl pantothenate was used instead of the polyoxyethylene (9) lauryl ether.

Example 136

The transdermal therapeutic preparations in the form of a gel were obtained in the same manner as in Example 132, except that 0.1 g of sucrose fatty acid ester (see Example 5) was used instead of the polyoxyethylene (9) lauryl ether.

Example 137

A 5 g amount of diltiazem hydrochloride was suspended in 1 ml of purified water at room temperature and 1.25 g of sodium polyoxyethylene (4) lauryl ether sulfate was added thereto while stirring. Thus, a suspension was obtained.

Then, 22.5 g of polyethylene glycol (400), 2.5 g of polyethylene glycol (600), and 25 g of polyethylene glycol (4000) were mixed or incorporated together and the suspension obtained above was gradually added thereto. Thus, the desired transdermal therapeutic preparations in the form of an ointment were prepared.

Example 138

The transdermal therapeutic preparations in the form of an ointment were obtained in the same manner as in Example 137, except that the amount of sodium polyoxyethylene (4) lauryl ether sulfate was changed to 0.625 g and 0.625 g of polyoxyethylene (9) lauryl ether was further used.

Example 139

The transdermal therapeutic preparations in the form of an ointment were obtained in the same manner as in Example 137, except that 0.5 g of salbutamol hemisulfate was used instead of the diltiazem hydrochloride.

Example 140

The transdermal therapeutic preparations in the form of an ointment were obtained in the same manner as in Example 139, except that 1.25 g of 2-undecyl-N-carboxymethyl-N-hydroxyethyl imidazolinium betaine was used instead of the sodium polyoxyethylene (4) lauryl ether sulfate.

Example 141

The transdermal therapeutic preparations in the form of an ointment were obtained in the same manner as in Example 139, except that the amount of sodium polyoxyethylene (4) lauryl ether sulfate was changed to 0.625 g and 0.625 g of polyoxyethylene (9) lauryl ether was further used.

Example 142

The transdermal therapeutic preparations in the form of an ointment were obtained in the same manner as in Example 140, except that the amount of 2-undecyl-N-carboxymethyl-N-hydroxyethyl imidazolinium betaine was changed to 0.625 g and 0.625 g of polyoxyethylene (9) lauryl ether was further used.

Example 143

The transdermal therapeutic preparations in the form of an ointment were obtained in the same manner as in Example 137, except that 1 g of carbinoxamine maleate was used instead of the diltiazem hydrochloride.

Example 144

The transdermal therapeutic preparations in the form of an ointment were obtained in the same manner as in Example 137, except that 6 g of disodium cromoglycate was used instead of the diltizaem hydrochloride and 1.25 g of triethanolamine was used instead of the sodium polyoxyethylene (4) lauryl ether sulfate.

Example 145

The transdermal therapeutic preparations in the form of an ointment were obtained in the same manner as in Example 144, except that 1.25 g of choline chloride was used instead of the triethanolamine.

Example 146

The transdermal therapeutic preparations in the form of an ointment were obtained in the same manner as in Example 144, except that 1.25 g of dimethylamino acetic acid betaine was used instead of the triethanolamine.

Example 147

A mixture of 10 g of purified water, 7.5 g of glycerol, and 15 g of kaolin was sufficiently stirred and 3 g of polyvinyl alcohol (NH-18 available from NIHON GOUSEI KAGAKU KOGYO K.K., Japan) was gradually added thereto and mixed together, while stirring.

To the resultant mixture, 7.5 g of diltiazem hydrochloride, 2.5 g of 2-undecyl-N-carboxymethyl-N-hydroxyethyl imidazolinium betaine, and 2.5 g of polyoxyethylene (9) lauryl ether were added and thoroughly mixed together. Thereafter, 2 ml of a 5% aqueous boric acid solution was gradually added thereto.

The mixture was spread or coated onto non-woven fabrics having a size of 50 cm x 50 cm square.

Polyethylene films are applied onto the coated surfaces. Thus, the desired transdermal therapeutic preparations in the form of a poultice were obtained.

Example 148

The transdermal therapeutic preparations in the form of a poultice were obtained in the same manner as in Example 147, except that 2.5 g of sodium polyoxyethylene (4) lauryl ether sulfate was used instead of 2-undecyl-N-carboxymethyl-N-hydroxyethyl imidazolinium betaine.

Example 149

The transdermal therapeutic preparations in the form of a poultice were obtained in the same manner as in Example 147, except that 2.5 g of sodium polyoxyethylene (4) lauryl ether phosphate was used instead of 2-undecyl-N-carboxymethyl-N-hydroxyethyl imidazolinium betaine.

Example 150

The transdermal therapeutic preparations in the form of a poultice were obtained in the same manner as in Example 148, except that 1.5 g of bunitrolol hydrochloride was used instead of the diltiazem hydrochloride.

Example 151

The transdermal therapeutic preparations in the form of a poultice were obtained in the same manner as in Example 150, except that 2.5 g of sodium polyoxyethylene (4) lauryl ether phosphate was used instead of the sodium polyoxyethylene (4) lauryl ether sulfate.

Example 152

The transdermal therapeutic preparations in the form of a poultice were obtained in the same manner as in Example 147, except that 1.0 g of salbutamol hemisulfate was used instead of the diltiazem hydrochloride.

Example 153

The transdermal therapeutic preparations in the form of a poultice were obtained in the same manner as in Example 152, except that 2.5 g of sodium polyoxyethylene (4) lauryl ether sulfate was used instead of the 2-undecyl-N-carboxymethyl-N-hydroxyethyl imidazolinium betaine.

Example 154

The transdermal therapeutic preparations in the form of a poultice were obtained in the same manner as in Example 152, except that 2.5 g of sodium lauroyl sarcosinate was used instead of the 2-undecyl-N-carboxymethyl-N-hydroxyethyl imidazolinium betaine.

Example 155

The transdermal therapeutic preparations in the form of a poultice were obtained in the same manner as in Example 152, except that 0.15 g of tulobuterol hydrochloride was used instead of the salbutamol hemisulfate.

Example 156

The transdermal therapeutic preparations in the form of a poultice were obtained in the same manner as in Example 155, except that 2.5 g of sodium polyoxyethylene (4) lauryl ether sulfate was used instead of the 2-undecyl-N-carboxymethyl-N-hydroxyethyl imidazolinium betaine.

Example 157

The transdermal therapeutic preparations in the form of a poultice were obtained in the same manner as in Example 147, except that 6.5 g of disodium cromoglycate was used instead of the diltiazem hydrochloride.

Example 158

The transdermal therapeutic preparations in the form of a poultice were obtained in the same manner as in Example 157, except that 2.5 g of triethanolamine was used instead of the 2-undecyl-N-carboxymethyl-N-hydroxyethyl imidazolinium betaine.

Example 159

The transdermal therapeutic preparations in the form of a poultice were obtained in the same manner as in Example 157, except that 2.5 g of choline chloride was used instead of the 2-undecyl-N-carboxymethyl-N-hydroxyethyl imidazolinium betaine.

Comparative Example 1

The transdermal therapeutic preparations in the form of a film were obtained in the same manner as in Example 1, except that the dehydrocholic acid was not used.

Comparative Example 2

The transdermal therapeutic preparations in the form of a film were obtained in the same manner as in Example 30, except that the sodium polyoxyethylene (4) lauryl ether phosphate was not used.

Comparative Example 3

The transdermal therapeutic preparations in the form of a film were obtained in the same manner as in Example 35, except that the sodium polyoxyethylene (4) lauryl ether sulfate was not used.

Comparative Example 4

The transdermal therapeutic preparations in the form of a film were obtained in the same manner as in Example 38, except that the 2-undecyl-N-carboxymethyl-N-hydroxyethyl imidazolinium betaine was not used.

Comparative Example 5

The transdermal therapeutic preparations in the form of a film were obtained in the same manner as in Example 57, except that the sodium polyoxyethylene (4) lauryl ether phosphate was not used.

Comparative Example 6

The transdermal therapeutic preparations in the form of a film were obtained in the same manner as in Example 63, except that the sodium polyoxyethylene (4) lauryl ether sulfate was not used.

Comparative Example 7

The transdermal therapeutic preparations in the form of a film were obtained in the same manner as in Example 76, except that the sodium polyoxyethylene (4) lauryl ether sulfate was not used.

Comparative Example 8

The transdermal therapeutic preparations in the form of a film were obtained in the same manner as in Example 81, except that the sodium polyoxyethylene (4) lauryl ether sulfate was not used.

Comparative Example 9

The transdermal therapeutic preparations in the form of a film were obtained in the same manner as in Example 90, except that the triethanolamine was not used.

Comparative Example 10

The transdermal therapeutic preparations in the form of a gel were obtained in the same manner as in Example 108, except that the sodium polyoxyethylene (4) lauryl ether phosphate and polyoxyethylene (9) lauryl ether were not used.

Comparative Example 11

The transdermal therapeutic preparations in the form of a gel were obtained in the same manner as in Example 115, except that the sodium polyoxyethylene (4) lauryl ether phosphate and polyoxyethylene (9) lauryl ether were not used.

Comparative Example 12

The transdermal therapeutic preparations in the form of a gel were obtained in the same manner as in Example 120, except that the sodium polyoxyethylene (4) lauryl ether phosphate and polyoxyethylene (9) lauryl ether were not used.

Comparative Example 13

The transdermal therapeutic preparations in the form of a gel were obtained in the same manner as in Example 124, except that the sodium polyoxyethylene (4) lauryl ether phosphate and polyoxyethylene (9) lauryl ether were not used.

Comparative Example 14

The transdermal therapeutic preparations in the form of a gel were obtained in the same manner as in Example 129, except that the triethanolamine and polyoxyethylene (9) lauryl ether were not used.

Comparative Example 15

The transdermal therapeutic preparations in the form of an ointment were obtained in the same manner as in Example 137, except that the sodium polyoxyethylene (4) lauryl ether sulfate was not used.

Comparative Example 16

The transdermal therapeutic preparations in the form of an ointment were obtained in the same manner as in Example 139, except that the sodium polyoxyethylene (4) lauryl ether sulfate was not used.

Comparative Example 17

The transdermal therapeutic preparations in the form of an ointment were obtained in the same manner as in Example 144, except that the triethanolamine was not used.

Comparative Example 18

The transdermal therapeutic preparations in the form of a poultice were obtained in the same manner as in Example 147, except that the 2-undecyl-N-carboxymethyl-N-hydroxyethyl imidazolinium betaine and polyoxyethylene (9) lauryl ether were not used.

Comparative Example 19

The transdermal therapeutic preparations in the form of a poultice were obtained in the same manner as in Example 152, except that the 2-undecyl-N-carboxymethyl-N-hydroxyethyl imidazolinium betaine and polyoxyethylene (9) lauryl ether were not used.

Comparative Example 20

The transdermal therapeutic preparations in the form of a poultice were obtained in the same manner as in Example 155, except that the 2-undecyl-N-carboxymethyl-N-hydroxyethyl imidazolium betaine and polyoxyethylene (9) lauryl ether were not used.

Comparative Example 21

The transdermal therapeutic preparations in the form of a poultice were obtained in the same manner as in Example 157, except that the 2-undecyl-N-carboxymethyl-N-hydroxyethyl imidazolinium betaine and polyoxyethylene (9) lauryl ether were not used.

Evaluation Test 1

The transdermal therapeutics preparations in the form of a film, a gel, an ointment, and a poultice prepared above were evaluated by using albino male rabbits having a body weight of about 3 kg. The hair on the abdominal skin of the rabbit was shaved on the previous day. Then, the next day, the sample preparations listed in Table 1 were applied to the shaved portions of the skins. After applying the sample preparations, samples of blood were taken from the aulicular vein at a predetermined time interval and the plasma was recovered therefrom in a conventional manner. The content of the active compound in the plasma was determined by means of high performance liquid chromatography.

The results are as shown in Table 1.

Evaluation Test 2

The transdermal therapeutic preparations in the form of a film, a gel, an ointment, and a poultice prepared above were evaluated by using Wistar male rats (8 weeks aged).

The hair on the abdominal skin of rats were shaved. Then, the next day, the sample preparations listed in Table 2 were applied to the shaved portions of the skins.

The rats were anesthetized 5 hrs after the administration, and the trachea was cannulated for artificial respiration.

Then, 300 µg/kg of serotonin, a broncho-constrictor was resistance was measured through the cannula connected to a pressure transducer.

The results are as shown in Table 2.

Evaluation Test 3

The film preparation prepared in Example 8 and the commercial tablet of diltiazem hydrochloride were evaluated by using albino male rabbits having a body weight of about 3 kg.

The film preparation was administrated in the same manner as in Evaluation Test 1, and the commercial tablet was orally administrated.

After the administration, blood samples were collected from auricular vein at the predetermined time intervals, and the plasma was recovered therefrom in a conventional manner. The content of diltiazem and deacetyl-metabolite in the plasma were determined by means of high-speed liquid chromatography.

The results are as shown in Table 3.

Evaluation Test 4

The film preparation prepared in Example 8 and the commercial tablet of diltiazem hydrochloride were evaluated by using albino male rabbits having a body weight of 3 kg.

The rabbits were anesthetized and cannulated to the abdominal aorta through the femoral artery.

Seven days after the cannulation, the film preparation was administrated in the same manner as in Evaluation Test 1, and the commercial tablet was orally administrated.

After the administration, blood pressure was measured by means of a pressure transducer connected to the cannula.

The results are as shown in Table 4.

## Table 1

| Example No.[1] | Type | Active Component | Concentration (µg/ml) in Plasma | | | | |
|---|---|---|---|---|---|---|---|
| | | | 1 hr | 2 hr | 4 hr | 7 hr | 24 hr |
| Ex. 1 | Film | Diltiazem hydrochloride | 0.092 | 0.080 | 0.077 | 0.071 | 0.036 |
| Ex. 2 | " | " | 0.070 | 0.107 | 0.133 | 0.107 | 0.051 |
| Ex. 3 | " | " | 0.187 | 0.224 | 0.226 | 0.175 | 0.169 |
| Ex. 4 | " | " | 0.151 | 0.129 | 0.124 | 0.125 | 0.076 |
| Ex. 5 | " | " | 0.390 | 0.608 | 0.539 | 0.476 | 0.150 |
| Ex. 6 | " | " | 0.178 | 0.286 | 0.235 | 0.160 | 0.088 |
| Ex. 7 | " | " | 0.524 | 0.502 | 0.327 | 0.199 | 0.106 |
| Ex. 8 | " | " | 0.203 | 0.275 | 0.351 | 0.263 | 0.072 |
| Ex. 9 | " | " | 0.225 | 0.260 | 0.270 | 0.294 | 0.178 |
| Ex. 10 | " | " | 0.087 | 0.101 | 0.092 | 0.076 | 0.057 |
| Ex. 11 | " | " | 0.044 | 0.103 | 0.124 | 0.139 | 0.106 |
| Ex. 12 | " | " | 0.132 | 0.201 | 0.265 | 0.192 | 0.134 |
| Ex. 13 | " | " | 0.036 | 0.057 | 0.068 | 0.054 | 0.045 |
| Ex. 14 | " | " | 0.078 | 0.115 | 0.113 | 0.098 | 0.063 |
| Com. Ex. 1 | " | " | 0.008 | 0.013 | 0.015 | 0.011 | 0.005 |

Table 1 (Continued)

| Example No.[1] | Type | Active Component | Concentration (µg/ml) in Plasma | | | | |
|---|---|---|---|---|---|---|---|
| | | | 1 hr | 2 hr | 4 hr | 7 hr | 24 hr |
| Ex. 15 | Film | Diltiazem hydrochloride | 0.396 | 0.427 | 0.474 | 0.325 | 0.082 |
| Ex. 16 | " | " | 0.211 | 0.292 | 0.344 | 0.357 | 0.046 |
| Ex. 17 | " | " | 0.154 | 0.352 | 0.493 | 0.382 | 0.164 |
| Ex. 18 | " | " | 0.465 | 0.531 | 0.692 | 0.638 | 0.125 |
| Ex. 19 | " | " | 0.559 | 0.661 | 0.874 | 0.538 | 0.127 |
| Ex. 20 | " | " | 0.873 | 1.004 | 0.650 | 0.426 | 0.103 |
| Ex. 21 | " | " | 0.495 | 0.539 | 0.553 | 0.481 | 0.278 |
| Ex. 22 | " | " | 0.806 | 0.762 | 0.611 | 0.369 | 0.199 |
| Ex. 23 | " | " | 0.303 | 0.371 | 0.430 | 0.331 | 0.235 |
| Ex. 24 | " | " | 0.250 | 0.428 | 0.322 | 0.275 | 0.120 |
| Ex. 25 | " | " | 0.223 | 0.283 | 0.351 | 0.400 | 0.170 |
| Ex. 26 | " | " | 0.502 | 0.545 | 0.409 | 0.311 | 0.161 |
| Ex. 27 | " | " | 0.317 | 0.336 | 0.300 | 0.232 | 0.082 |
| Ex. 28 | " | " | 0.335 | 0.510 | 0.577 | 0.390 | 0.134 |
| Ex. 29 | " | " | 0.264 | 0.302 | 0.342 | 0.285 | 0.240 |
| Com. Ex. 1 | " | " | 0.008 | 0.013 | 0.015 | 0.011 | 0.005 |

0189861

Table 1 (Continued)

| Example No. [1] | Type | Active Component | Concentration (µg/ml) in Plasma | | | | |
|---|---|---|---|---|---|---|---|
| | | | 1 hr | 2 hr | 4 hr | 7 hr | 24 hr |
| Ex. 30 | Film | Acebutolol hydrochloride | 0.073 | 0.150 | 0.194 | 0.226 | 0.061 |
| Ex. 31 | " | " | 0.116 | 0.187 | 0.171 | 0.188 | 0.085 |
| Ex. 32 | " | " | 0.044 | 0.106 | 0.235 | 0.140 | 0.037 |
| Ex. 33 | " | " | 0.415 | 0.499 | 0.450 | 0.383 | 0.154 |
| Ex. 34 | " | " | 0.801 | 0.544 | 0.373 | 0.281 | 0.116 |
| Com. Ex. 2 | " | " | 0.007 | 0.012 | 0.054 | 0.035 | 0.007 |
| Ex. 35 | Film | Alprenolol hydrochloride | 0.062 | 0.087 | 0.103 | 0.094 | 0.040 |
| Ex. 36 | " | " | 0.049 | 0.078 | 0.050 | 0.055 | 0.033 |
| Ex. 37 | " | " | 0.040 | 0.095 | 0.129 | 0.162 | 0.062 |
| Com. Ex. 3 | " | " | 0.015 | 0.023 | 0.026 | 0.021 | 0.009 |
| Ex. 38 | Film | Metoprolol Tartrate | 0.112 | 0.150 | 0.128 | 0.077 | 0.019 |
| Ex. 39 | " | " | 0.028 | 0.048 | 0.057 | 0.071 | 0.068 |
| Ex. 40 | " | " | 0.439 | 0.291 | 0.164 | 0.113 | 0.042 |
| Com. Ex. 4 | " | " | 0.006 | 0.011 | 0.012 | 0.024 | 0.007 |

Table 1 (Continued)

| Example No. [*1] | Type | Active Component | Concentration (µg/ml) in Plasma | | | | |
|---|---|---|---|---|---|---|---|
| | | | 1 hr | 2 hr | 4 hr | 7 hr | 24 hr |
| Ex. 57 | Film | Ketamine hydrochloride | 0.226 | 0.301 | 0.547 | 0.452 | 0.098 |
| Ex. 58 | " | " | 0.274 | 0.499 | 0.475 | 0.596 | 0.160 |
| Ex. 59 | " | " | 1.375 | 1.633 | 1.702 | 1.489 | 0.504 |
| Com. Ex. 5 | " | " | 0.032 | 0.066 | 0.126 | 0.129 | 0.025 |
| Ex. 90 | Film | Disodium cromoglycate | 0.385 | 0.468 | 0.327 | 0.204 | 0.015 |
| Ex. 91 | " | " | 0.367 | 0.391 | 0.278 | 0.162 | 0.027 |
| Ex. 92 | " | " | 0.313 | 0.275 | 0.240 | 0.096 | 0.024 |
| Ex. 93 | " | " | 0.221 | 0.246 | 0.195 | 0.115 | 0.011 |
| Ex. 94 | " | " | 0.239 | 0.292 | 0.104 | 0.053 | 0.008 |
| Ex. 97 | " | " | 1.102 | 1.277 | 1.096 | 0.678 | 0.306 |
| Ex. 98 | " | " | 0.544 | 0.720 | 0.689 | 0.420 | 0.142 |
| Ex. 99 | " | " | 0.607 | 0.779 | 0.501 | 0.227 | 0.080 |
| Ex. 100 | " | " | 1.498 | 1.393 | 0.902 | 0.561 | 0.202 |
| Com. Ex. 9 | " | " | 0.004 | 0.007 | 0.010 | 0.012 | 0.004 |

Table 1 (Continued)

| Example No. [1] | Type | Active Component | Concentration (µg/ml) in Plasma | | | | |
|---|---|---|---|---|---|---|---|
| | | | 1 hr | 2 hr | 4 hr | 7 hr | 24 hr |
| Ex. 101 | Film | Disodium cromoglycate | 1.924 | 1.950 | 1.551 | 0.972 | 0.103 |
| Ex. 102 | " | " | 0.981 | 1.123 | 1.245 | 1.037 | 0.390 |
| Ex. 103 | " | " | 0.852 | 0.985 | 0.803 | 0.684 | 0.191 |
| Ex. 104 | " | " | 2.297 | 2.054 | 1.846 | 1.121 | 0.285 |
| Com. Ex. 9 | " | " | 0.004 | 0.007 | 0.010 | 0.012 | 0.004 |
| Ex. 108 | Gel | Diltiazem hydrochloride | 0.323 | 0.488 | 0.584 | 0.455 | 0.292 |
| Ex. 109 | " | " | 0.665 | 0.730 | 0.462 | 0.283 | 0.027 |
| Ex. 110 | " | " | 0.846 | 0.881 | 0.638 | 0.366 | 0.049 |
| Ex. 111 | " | " | 0.230 | 0.392 | 0.375 | 0.340 | 0.206 |
| Com. Ex. 10 | " | " | 0.019 | 0.025 | 0.022 | 0.020 | 0.005 |
| Ex. 129 | Gel | Disodium cromoglycate | 2.071 | 1.884 | 1.459 | 0.957 | 0.120 |
| Ex. 131 | " | " | 1.082 | 1.323 | 1.241 | 0.899 | 0.221 |
| Ex. 133 | " | " | 1.424 | 1.526 | 1.123 | 0.644 | 0.173 |
| Ex. 135 | " | " | 0.978 | 1.089 | 0.810 | 0.511 | 0.074 |
| Com. Ex. 14 | " | " | 0.009 | 0.025 | 0.021 | 0.018 | 0.004 |

Table 1 (Continued)

| Example No. [*1] | Type | Active Component | Concentration (μg/ml) in Plasma | | | | |
|---|---|---|---|---|---|---|---|
| | | | 1 hr | 2 hr | 4 hr | 7 hr | 24 hr |
| Ex. 137 | Ointment | Diltiazem hydrochloride | 0.082 | 0.118 | 0.131 | 0.119 | 0.063 |
| Ex. 138 | " | " | 0.141 | 0.223 | 0.314 | 0.350 | 0.105 |
| Com. Ex. 15 | " | " | 0.003 | 0.007 | 0.010 | 0.011 | 0.004 |
| Ex. 144 | Ointment | Disodium cromoglycate | 0.119 | 0.215 | 0.292 | 0.204 | 0.080 |
| Ex. 145 | " | " | 0.297 | 0.632 | 0.705 | 0.563 | 0.112 |
| Ex. 146 | " | " | 0.090 | 0.181 | 0.213 | 0.175 | 0.037 |
| Com. Ex. 17 | " | " | 0.003 | 0.006 | 0.006 | 0.009 | 0.003 |
| Ex. 148 | Poultice | Diltiazem hydrochloride | 0.114 | 0.180 | 0.227 | 0.232 | 0.150 |
| Ex. 149 | " | " | 0.052 | 0.079 | 0.089 | 0.077 | 0.051 |
| Com. Ex. 18 | " | " | 0.009 | 0.014 | 0.014 | 0.013 | 0.006 |
| Ex. 158 | Poultice | Disodium cromoglycate | 0.315 | 0.691 | 0.868 | 0.471 | 0.102 |
| Ex. 159 | " | " | 0.261 | 0.438 | 0.590 | 0.545 | 0.154 |
| Com. Ex. 21 | " | " | 0.004 | 0.007 | 0.008 | 0.007 | 0.005 |

*1: Ex. = Example, Com. Ex. = Comparative Example

## Table 2

| Example No. [1] | Type | Active Component | Increase in Airway Resistance $(mmH_2O)$ |
|---|---|---|---|
| Ex. 63 | Film | Salbutamol hemisulfate | 39.2 |
| Ex. 64 | " | " | 53.9 |
| Ex. 65 | " | " | 22.1 |
| Ex. 66 | " | " | 49.5 |
| Ex. 67 | " | " | 57.8 |
| Ex. 69 | " | " | 32.3 |
| Ex. 71 | " | " | 63.4 |
| Ex. 72 | " | " | 31.3 |
| Ex. 73 | " | " | 32.9 |
| Ex. 74 | " | " | 18.1 |
| Ex. 75 | " | " | 51.6 |
| Com. Ex. 6 | " | " | 86.5 |

Table 2 (Continued)

| Example No. [1] | Type | Active Component | Increase in Airway Resistance (mm$H_2O$) |
|---|---|---|---|
| Ex. 76 | Film | Terbutaline sulfate | 56.3 |
| Ex. 77 | " | " | 40.0 |
| Ex. 78 | " | " | 53.8 |
| Ex. 79 | " | " | 47.5 |
| Ex. 80 | " | " | 33.2 |
| Com. Ex. 7 | " | " | 91.7 |
| Ex. 81 | " | Turobuterol hydrochloride | 60.5 |
| Ex. 82 | " | " | 64.7 |
| Ex. 83 | " | " | 70.6 |
| Ex. 84 | " | " | 42.1 |
| Com. Ex. 8 | " | " | 98.4 |

0189861

Table 2 (Continued)

| Example No. *1 | Type | Active Component | Increase in Airway Resistance (mmH$_2$O) |
|---|---|---|---|
| Ex. 115 | Gel | Salbutamol hemisulfate | 30.8 |
| Ex. 116 | " | " | 27.5 |
| Ex. 117 | " | " | 14.1 |
| Ex. 118 | " | " | 37.4 |
| Ex. 119 | " | " | 31.3 |
| Com. Ex. 11 | " | " | 79.9 |
| Ex. 120 | Gel | Terbutaline sulfate | 43.5 |
| Ex. 121 | " | " | 41.6 |
| Ex. 122 | " | " | 29.3 |
| Ex. 123 | " | " | 33.8 |
| Com. Ex. 12 | " | " | 82.0 |
| Ex. 124 | Gel | Tulobuterol hydrochloride | 63.2 |
| Ex. 125 | " | " | 40.5 |
| Ex. 126 | " | " | 49.7 |
| Com. Ex. 13 | " | " | 82.1 |

0189861

Table 2 (Continued)

| Example No. [1] | Type | Active Component | Increase in Airway Resistance $(mmH_2O)$ |
|---|---|---|---|
| Ex. 140 | Ointment | Salbutamol hemisulfate | 38.9 |
| Ex. 141 | " | " | 48.2 |
| Ex. 142 | " | " | 32.0 |
| Com. Ex. 16 | " | " | 96.6 |
| Ex. 152 | Poultice | " | 25.8 |
| Ex. 153 | " | " | 37.3 |
| Comp. Ex. 19 | " | " | 91.9 |
| Ex. 155 | Poultice | " | 55.7 |
| Ex. 156 | " | " | 51.4 |
| Com. Ex. 20 | " | " | 85.3 |

[1]: Ex.=Example, Com. Ex.=Comparative Example

0189861

Table 3

| Example No. | | Concentration (μg/ml) in Plasma | | | | | | |
|---|---|---|---|---|---|---|---|---|
| | | 0.5 hr | 1 hr | 2 hr | 4 hr | 7 hr | 10 hr | 24 hr |
| Example 8 (Film) | Diltiazem | n.d.[*1] | 0.203 | 0.275 | 0.351 | 0.263 | n.d. | 0.072 |
| | Deacetyl metabolite | n.d. | 0.035 | 0.051 | 0.072 | 0.065 | n.d. | 0.034 |
| Commercial Tablet · | Diltiazem | 0.399 | 0.241 | 0.176 | 0.124 | 0.122 | 0.033 | n.d. |
| | Deacetyl metabolite | 0.098 | 0.067 | 0.072 | 0.092 | 0.150 | 0.124 | n.d. |

*1: Not Determined

Table 4

| Example No. | Change of Mean Blood Pressure (mmHg) | | | | | | | |
|---|---|---|---|---|---|---|---|---|
| | 0.25 hr | 0.5 hr | 1 hr | 2 hr | 4 hr | 7 hr | 10 hr | 24 hr |
| Example 8 (Film) | − 1.0 | − 1.2 | − 1.5 | −12.7 | −17.3 | −15.7 | −19.0 | − 8.3 |
| Commercial Tablet | −16.7 | −17.7 | −15.2 | −13.3· | −11.0 | − 9.2 | − 9.6 | − 5.1 |
| Non-Administration (Control) | − 1.2 | − 0.8 | − 1.3 | − 2.7 | − 5.7 | − 6.0 | − 6.2 | − 3.2 |

0189861

CLAIMS

1. A percutaneous absorption accelerator for ionic water-soluble medicines comprising at least one member selected from the group consisting of ionic oil-soluble substances and their salts, amphoteric surfactants, and nonionic substances.

2. A percutaneous absorption accelerator as claimed in claim 1, wherein said ionic water-soluble medicines are cationic water-soluble medicines having a cationic group, a pKa of 7.0 or more, and a solubility such that 1 g of the medicine can be dissolved in an amount of less than 10 ml of water at 25°C.

3. A percutaneous absorption accelerator as claimed in claim 2, wherein said cationic water-soluble medicines are acebutolol hydrochloride, alprenolol hydrochloride, metoprolol tartrate, indenolol hydro-chloride, oxprenolol hydrochloride, bufetolol hydro-chloride, bunitrolol hydrochloride, trimetazidine hydrochloride, disopyramide phosphate, procainamide hydrochloride, diltiazem hydrochloride, guanethidine sulfate, betanidine sulfate, mecamylamine hydrochloride, norfenefrine hydrochloride, etilefrine hydrochloride, octopamine hydrochloride, fluphenazine dihydrochloride, amitriptyline hydrochloride, imipramine hydrochloride, opipramol hydrochloride, chlorpromazine hydrochloride, tramadol hydrochloride, benzydamine hydrochloride, acamylophenine hydrochloride, trospium chloride, valethamate bromide, scopolamine butylbromide, prifinium bromide, diponium beomide, N-methylscopolamine methyl-sulfate, ketamine hydrochloride, tetracaine hydro-chloride, cocaine hydrochloride, dibucaine hydrochloride, procaine hydrochloride, prilocaine hydrochloride, lidocaine hydrochloride, salbutamol hemisulfate, terbutaline sulfate, tulobuterol hydrochloride, isoproterenol hydrochloride, clorprenaline hydrochloride, orciprenaline sulfate, methoxyphenamine hydrochloride, alloclamide hydrochloride, oxeladin citrate, carbetapentane citrate, ephedrine hydrochloride, cloperastine hydrochloride, codeine phosphate, dihydrocodeine phosphate, alimemazine tartrate, triprolidine hydrochloride, carbinoxamine maleate, chlorpheniramine maleate, diphenylpyraline hydrochloride, promethazine hydrochloride, and homochlorcyclizine hydrochloride.

4.    A percutaneous absorption accelerator as claimed in claim 1, wherein said ionic water-soluble medicines are anionic water-soluble medicines having an anionic gorup, a pKa of 4.5 or less, and a solubility such that 1 g of the medicine can be dissolved in an amount of less than 10 ml of water at 25°C.

5.    A percutaneous absorption accelerator as claimed in claim 4, wherein said anionic water-soluble medicines are disodium crymoglycate, sodium tranilast, dipotassium clorazepate, dexamethasone sodium phosphate, and betamethasone disodium phosphate.

6.    A percutaneous absorption accelerator as claimed in claim 2, wherein said ionic oil-soluble substances are acidic oil-soluble substances and their salts.

7.    A percutaneous absorption accelerator as claimed in claim 6, wherein said acidic oil-soluble substances and their salts are cholic acid derivatives and their salts, ethacrynic acid and their salt, and anionic surfactants.

8.    A percutaneous absorption accelerator as claimed in claim 4, wherein said ionic oil-soluble substances and their salts are basic oil-soluble substances and their salts.

9.    A percutaneous absorption accelerator as claimed in claim 8, wherein said basic oil-soluble substances and their salts are alkanol amines and their salts, alkyldiamines, and choline and its salts.

10.    A percutaneous absorption accelerator as claimed in claim 1, wherein said amphoteric surfactants are betaine type amphoteric surfactants, amino acid type amphoteric surfactants, sulfonic acid type amphoteric surfactants, and sulfuric acid ester type amphoteric surfactants.

11. A percutaneous absorption accelerator as claimed in claim 1, wherein said nonionic substances are pantothenic acid derivatives, p-aminobenzoic acid derivatives, sucrose fatty acid esters, polyoxyethylene sorbitan fatty acid esters, and polyoxyethylene alkyl ethers.

12. Pharmaceutical preparation for the percutaneous administration, comprising a ionic water-soluble medicine, characterized by additionally comprising a percutaneous absorption accelerator as claimed in any of the claims 1 to 11, wherein said accelerator preferably is present in an amount of 2 to 6000 parts by wt., based on 100 parts by wt. of the ionic medicine.